# EUROPEAN PATENT APPLICATION

(11) **EP 4 177 252 A2**
(43) Date of publication of application: **10.05.2023**
(21) Application number: 22198312.5
(22) Date of filing: 10.07.2020
(51) Int. Cl.: C07D 413/12, C07D 231/20, C07D 261/04, C07C 43/225

(54) **PROCESS AND INTERMEDIATES FOR THE PREPARATION OF PYROXASULFONE, FENOXASULFONE AND VARIOUS SULFONE ANALOGS OF 5,5-DIMETHYL-4H-1,2-OXAZOLE**

(62) Divisional of application: 20185255.5
(71) Applicant: Adama Agan Ltd., 7710201 Ashdod (IL)
(72) Inventor: Barda, Yaniv, Rehovot (IL); Papo, Nitsan, Kfar-Saba (IL); Recsei, Carl, Tel Aviv (IL)
(74) Representative: Balder IP Law, S.L.

(57) **Abstract**

The invention relates to a process for preparing immediate precursors for pyroxasulfone and fenoxasulfone of the formula (I) and (I'). 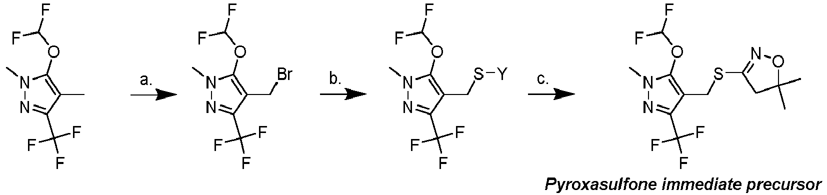 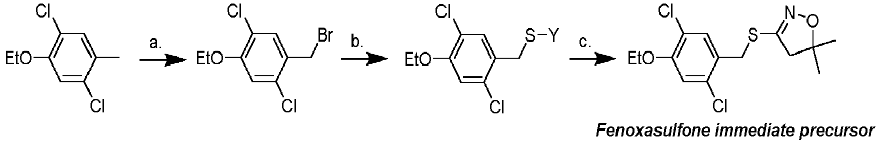 The pyroxasulfone or fenoxasulfone immediate precursor is synthesized from the arylmethyl bromide by first forming a carbon-sulfur bond at the 3-position of a 2-isoxazoline through displacement of a leaving group at the 3-position (particularly acid derived residues such as phosphoryl, sulfanyl, halo, cyano or carboxyl groups) by an appropriate thionating reagent. The resulting adducts, which is a S-protected 3-thio-2-isoxazoline, may be treated with base to remove the protecting group, to reveal a thiol or thiolate which may subsequently react with the arylmethyl bromide to form the Pyroxasulfone or Fenoxasulfone immediate precursor.

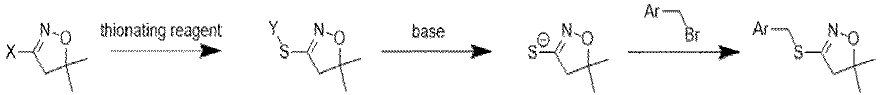

## Description

### TECHNICAL FIELD OF THE INVENTION

The present invention relates to a process for producing immediate precursors for the preparation of pyroxasulfone, fenoxasulfone and various sulfone analogs of 5,5-dimethyl-4H-1,2-oxazole.

### BACKGROUND OF THE INVENTION

Pyroxasulfone, having the chemical name 3-(5-(difluoromethoxy)-1-methyl-3-(trifluoromethyl)-1*H*-pyrazol-4-yl]methanesulfonyl)-5,5-dimethyl-4,5-dihydro-1,2-oxazole, has the following structural Formula (1):

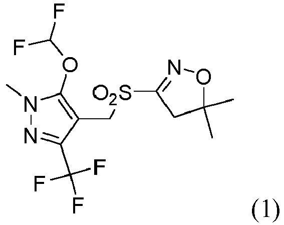

Pyroxasulfone belongs to the class of 3-([(hetero)aryl]methanesulfonyl)-4,5-dihydro-1,2-oxazoles and is used as an herbicide, in particular for pre-emergence control of annual grasses and some broad-leaved weeds in maize, soy beans, wheat and other crops. Pyroxasulfone's mode of action affects apical meristems and coleoptile development and inhibits the biosynthesis of very-long-chain fatty acids in plants with excellent herbicidal activity against grass and broadleaf weeds at lower application rates compared with other commercial herbicides. In fields of genetically modified crops, pyroxasulfone controlled weeds were resistant to non-selective herbicides. Pyroxasulfone has been classified in the Herbicide Resistance Action Committee Group K3.

Pyroxasulfone was first disclosed by Kumiai Chemical Industry Co., Ltd. and Ihara Chemical Industry Co., Ltd. in WO 2002/062770. It is available on the marketplace as water dispersible granule

Fenoxasulfone, having the chemical name 3-[(2,5-dichloro-4-ethoxyphenyl)methylsulfonyl]-5,5-dimethyl-4H-1,2-oxazole, has the following structural Formula (2):

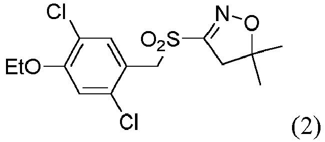

Fenoxasulfone and pyroxasulfone share the same moiety: a 5,5-dimethyl-4H-1,2-oxazole attached through the 3-position to an arylmethyl sulfone and is used as an herbicide, in particular for pre-emergence control of grass and broad-leaved weeds. Studies suggest that fenoxasulfone is a potent inhibitor of plant VLCFAEs (very-long-chain fatty acids elongase (VLCFAE)) and should be categorized within the K3 group of the Herbicide Resistance Action Committee. VLCFAE activity of recombinant Fatty Acid Elongation 1 (FAE1) of Arabidopsis, was inhibited by fenoxasulfone in a time-dependent manner, which has been shown in the inhibition of VLCFAEs by other well-known VLCFAE-inhibiting herbicides. On the other hand, VLCFAE activity of the microsomal fraction of etiolated barnyard millet seedlings, was inhibited by fenoxasulfone in a time-independent manner. This time-independent inhibition raised a new inhibition mechanism of VLCFAE by fenoxasulfone likely with pyroxasulfone, which is classified in the same chemical class as fenoxasulfone, as has been showed in Journal of Pesticide Science, 2011, Vol. 36 Issue 3, p357-362.

Fenoxasulfone is a novel rice herbicide that was discovered and developed by Kumiai Chemical Industry Co., Ltd. It displays excellent herbicidal activity against *Echinochloa* spp. and other annual weeds at 150-200 g a.i./ha with long residual activity and has a favorable toxicological, ecotoxicological, and environmental profile. Fenoxasulfone's mode of action was investigated, and it has been shown to inhibit the biosynthesis of very-long-chain fatty acids in plants. Fenoxasulfone was registered in Japan in 2014, and various products containing fenoxasulfone have been launched. With its high efficacy and long residual activity, it is estimated that fenoxasulfone will contribute to efficient food production in the future, as cited from J Pestic Sci, 2019 Jul 25; 44(4): 282-289.

Additional herbicides which contain the 5,5-dimethyl-4H-1,2-oxazole moiety, have been reported in WO 2010/145992 and WO 2011/018486 including, but not limited to:

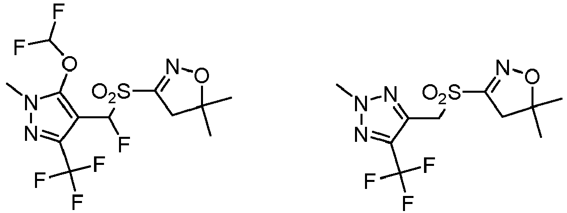

Pyroxasulfone's method of preparation was described in several patent applications namely WO 2006/068092 in the name of Ihara, WO 2004/013106 in the name of Kumiai and Ihara and WO 2005/095352 in the name of Ihara.

In the Pyroxasulfone synthetic route described by (WO 2004/013106 and WO 2016/042435), the bromination reaction was conducted with light irradiation. No reports exist of a non-photochemical transformation of formula (III) to formula (II). Bromination of formula (II) at the benzylic position without the need of light irradiation, described in the present invention, has benefits and advantages in industrial processes compared to a light-dependent reaction.

The present invention discloses process for the synthesis of immediate precursors for producing pyroxasulfone, fenoxasulfone and various sulfone analogs of 5,5-dimethyl-4H-1,2-oxazole.

### SUMMARY OF THE INVENTION

The present invention provides a process for preparing compounds of the formulae (I) or (I') 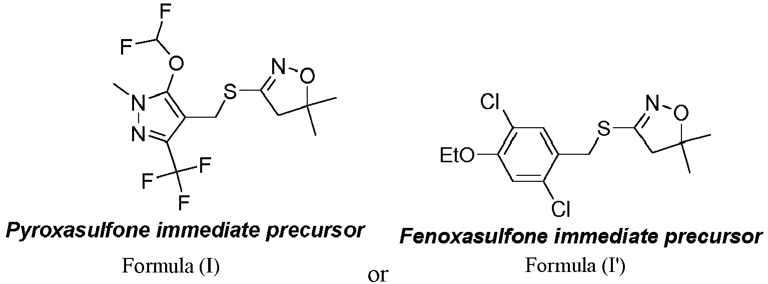 which comprises the following steps:
a) bromination of a starting material selected from the following compounds: 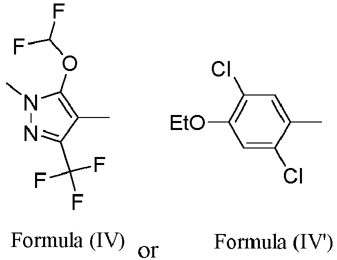 to obtain the following compounds: 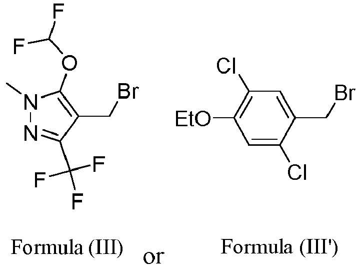 respectively;
b) thionation of the resulting compound of step a with a thionating agent to obtain the following compounds: 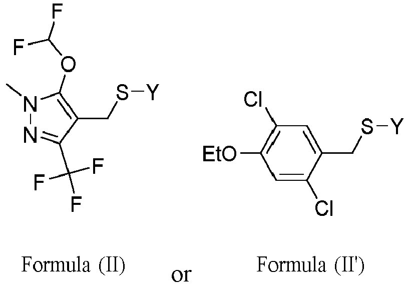 respectively,
   wherein Y is a protecting group;
c) substitution of the resulting thiol or thiolate created from a formulae (II) or (II') compounds of step b with an 2-isoxazoline moiety to yield the compound of formula (I).

The present invention also provides a process for preparing a compound of formulae (III) or (III') 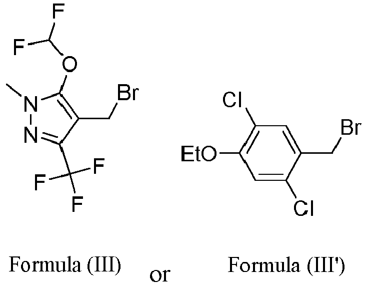 which comprises a brominating compounds of formulae (IV) or (IV') respectively: 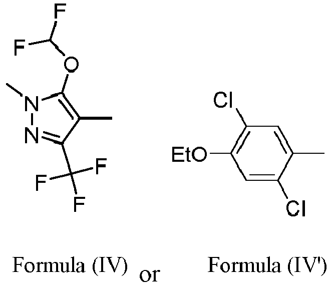 with a brominating reagent in the absence of light irradiation.

The present invention further provides a process for preparing pyroxasulfone or fenoxasulfone immediate precursors of formulae (I) or (I') and various sulfone analogs of 5,5-dimethyl-4H-1,2-oxazole, which comprises the following steps:
d) reacting an isoxazole of Formula (V) with a thionating reagent to create an S-substituted thioisoxazole of Formula (VI); 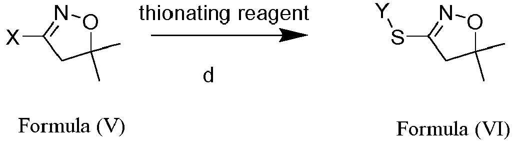 wherein X is a leaving group and Y is a protecting group;
e) substitution of the resulting thiol or thiolate created from formula (VI) compounds of step d with arylmethyl-bromide compounds of formula (VII) to yield the compound of formula (VIII)

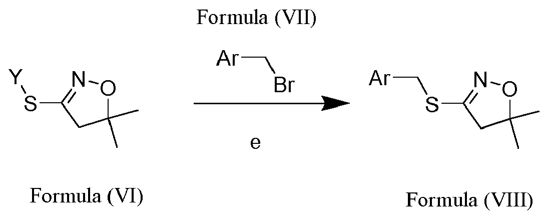

### DETAILED DESCRIPTION OF THE INVENTION

The present invention is a method to produce immediate precursors to pyroxasulfone, fenoxasulfone and immediate precursors for additional various sulfone analogs of 5,5-dimethyl-4H-1,2-oxazole, using a sequence of bromination, reaction with a thionating reagent to form a protected arylmethanethiol, then deprotection and subsequent formation of a new carbon-sulfur bond to install an isoxazoline moiety. The result is a substance which needs only to be oxidized at the sulfur to form the known herbicides pyroxasulfone, fenoxasulfone and various sulfone analogs of 5,5-dimethyl-4H-1,2-oxazole.

The invention is shown in Figure 1. 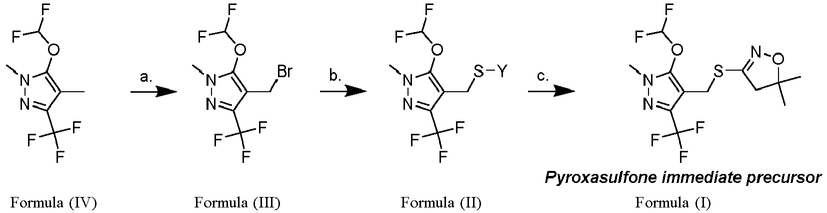 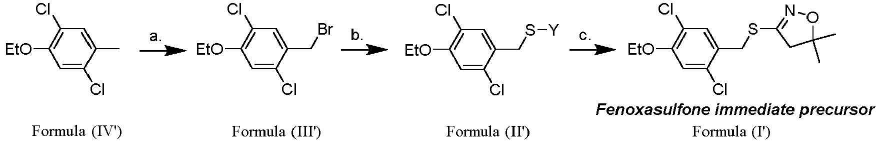 **Figure 1.** Invention overview.
Y = protecting group

Three synthetic steps characterize the route:
a. bromination, carried out under radical conditions.
b. thionating reagent (a sulfur nucleophile with a protecting group) is introduced, which substitutes the bromine atom.
c. the protecting group is removed, and the revealed thiol or thiolate reacts with a substituted isoxazoline bearing a leaving group at the 3-position.

The current invention can utilize a range of brominating reagents, including cheap and readily available hydrobromic acid in the presence of an oxidizer or elemental bromine. Also, the present invention can be utilized using radical initiators.

Also, an efficient synthesis of fenoxasulfone has not yet been reported. Therefore, there is an increasing need for an inexpensive, high yielding and efficient synthetic pathway towards fenoxasulfone.

The reaction of arylmethyl bromide compounds with thionating reagents is known, however a requirement for long reaction times (WO 2016/042435 A1), catalysts (WO 2009/129954 A2, WO 2016/042435 A1), prolonged heating (DE 10 200 506 306.6 A1, WO 2009/068170 A2) or the use of thionating reagents with difficult to remove or nonvolatile byproducts, such as urea (WO 2007/071900 A1) means that these processes have scope for improvement. Judicious choice of thionating reagent in this invention allows for the creation of volatile, organic-insoluble or highly water-soluble byproducts after deprotection, depending on the chemical properties and desired means of isolating the product.

The present invention permits the choice of a thionating reagent that gives volatile or insoluble byproducts, or which hydrolyze slowly, quickly or in the presence of a particular reagent according to the requirements of the operator.

The present invention allows very rapid substitution of the bromide with a thionating reagent, permitting extension of the invention to arylmethyl bromides which would otherwise be prone to hydrolysis or side reactions.

The use of a range of thionating reagents in step **b,** according to Fig. 1, allows the product isolation sequence to be simplified and improved, for example the use of a thioacetate salt as a thionating reagent results in a volatile ester byproduct in step **c** upon base-mediated deacylation of the formed S-arylmethyl thioacetate. Also, step **b** can be performed using nucleophilic catalysts such as tertiary amines.

The use of thionating reagents that create volatile or non-organic-soluble byproducts allows steps **b** and **c** to be telescoped into a unified process for the preparation of the immediate precursor to pyroxasulfone or fenoxasulfone in an organic solvent, uncontaminated with materials that would hinder the oxidation of these materials to give these herbicides, or which would interfere with the product isolation procedure.

Step c is carried out under basic conditions: suitable bases for generating the alkaline environment in the reaction mixture include alkali metal hydroxides, alkali metal hydrogencarbonates, alkali metal alkoxides, alkali metals hydride, organic bases and where applicable the corresponding alkaline earth bases. The amount of the base is in the range of 2:1 to 10:1 relative to the arymethanethiol starting material. Also, step **c** can be performed using Rongalite, tris-(2-carboxyethyl)phosphine or other inhibitors of oxidative disulfide formation and/or nucleophilic catalysts such as tertiary amines.

Pyroxasulfone and fenoxasulfone can be synthesized by reacting a thionating agent with the appropriate arylmethyl halide. A thionating reagent suitable for such a process is a nucleophilic, sulfur-containing reagent such as xanthate salts, thiocarbonyl compounds (except thiourea), thiosulfates, sulfinates, substituted thioureas and thioamides.

The thionating reagent reacts with the arylmethyl compound of formula (II) to produce an intermediate with the form Y-S-CH₂Ar, depicted in Figure **2**

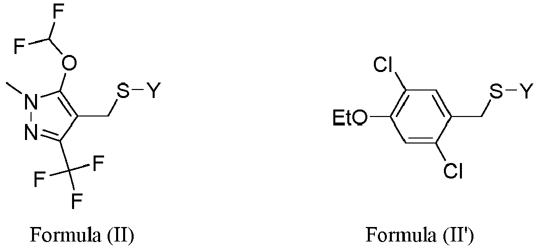

The thionating reagent reacts with the arylmethyl compound of formula (VII) to produce an intermediate with the form Y-S-CH₂Ar, depicted in Figure **2**

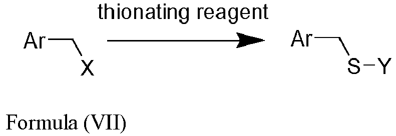

Wherein X is a halide and Y is a protecting group

**Figure 2.** Key intermediate with variable 'Y. '
and described in the table below.

| Thionating reagent | Y = | Numerical reference for Figures **3** and **4** |
|---|---|---|
| alkylxanthate salts | alkoxycarbonyl | 1 |
| dimethylthioformamide | *1-(N,N-*dimethylmethaniminium) | 2 |
| dithiooxamide | dithiooxamoyl | 3 |
| salts of thioacetic acid | acetyl | 4 |
| thiobenzamide | 1-(1-phenylmethiminium). | 5 |
| thiosulfate salt | sulfonyl salt | 6 |
| 1,3-dialkylthiourea | 1-(*N*,*N*-dialkyl(1-amino)methiminium) | 7 |

Figures **3** and **4** show the structures implied by the general formulas in the table.

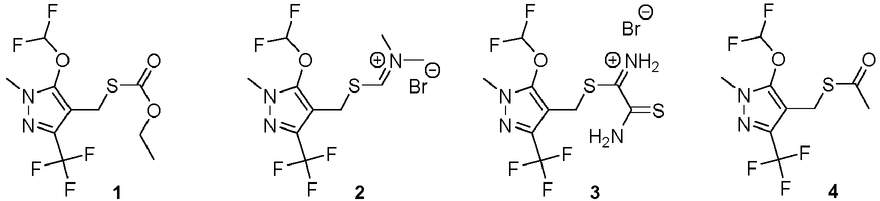

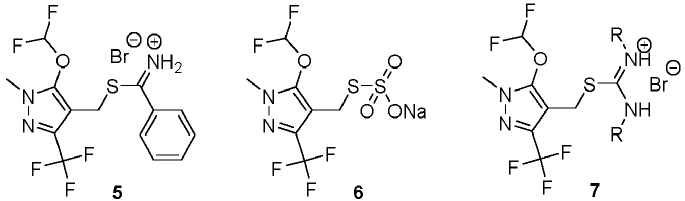

**Figure 3. Products of Step b. (Pyroxasulfone synthesis).**

R = alkyl.

Sodium is shown as a representative counterion to the sulfonyl salt.

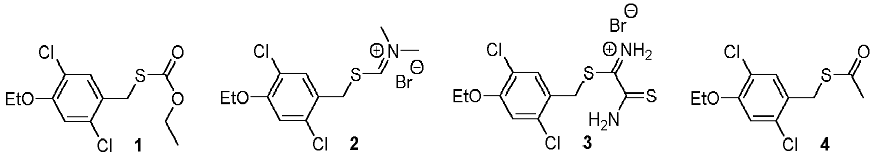

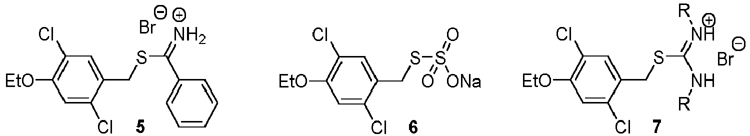

**Figure 4.** Products of Step b. (Fenoxasulfone synthesis).

R = alkyl.

Sodium is shown as a representative counterion to the sulfonyl salt.

A basic scheme to show the chemistry involved in Step c is shown in Figure **5.** A reactant of the form Y-S-CH₂Ar is first treated with a base to reveal a nucleophilic sulfide, which reacts with a 2-isoxazoline with a leaving group at the 3-position (particularly acid derived residues such as phosphoryl, sulfanyl, halo, cyano or carboxyl groups).

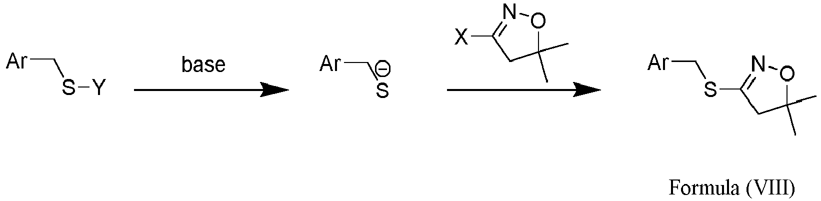

**Figure 5.** Detail of Step c.

Ar = aryl, X = leaving group

The substituted isoxazoline bearing a leaving group at the 3-position, is previously described and the process is well known (WO 2011/063843 A1 and WO 2006/038657 A1).

Step a of the present invention can be carried out without an initiator. Also, the reaction may generate radicals thermally without an initiator added.

Also, step **a** of the present invention can be chlorination instead of bromination.

Also, the present invention allows to combine Steps **b** and **c** in the following manner: the intermediate Y-S-CH₂Ar is generated from BrCH₂Ar and the thionating reagent and treated with base *in situ* to obtain the thiolate. The thiolate then reacts with a substituted isoxazoline bearing a leaving group at the 3-position to form the product.

In one aspect of the present invention is a process for preparing compounds of the formulae (I) or (I') 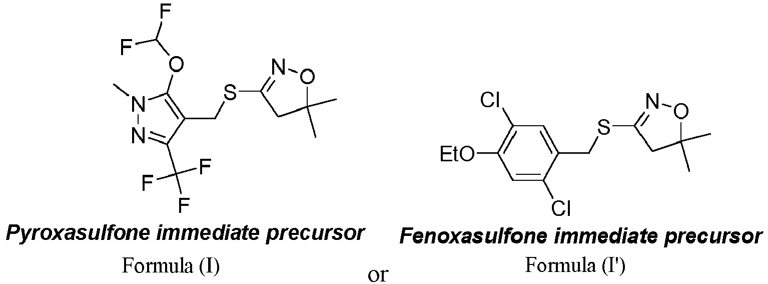 which comprises the following steps:
a) bromination of a starting material selected from the following compounds: 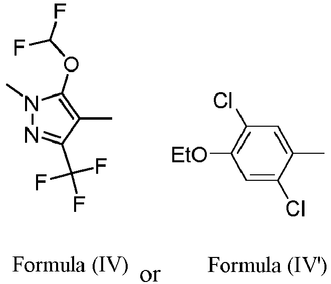 to obtain the following compounds: 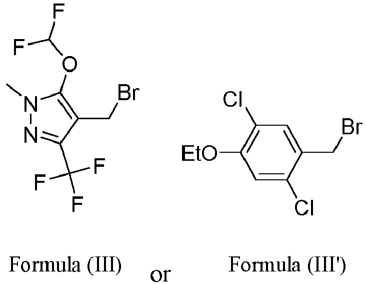 respectively;
b) thionation of the resulting compound of step a with a thionating agent to obtain the following compounds: 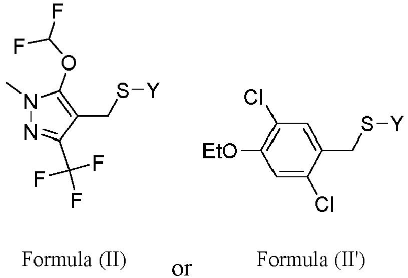 respectively;
c) substitution of the resulting thiol or thiolate created from a formulae (II) or (II') compounds of step b with an 2-isoxazoline moiety to yield the compound of formula (I).

In yet another aspect of the present invention is a process wherein the process is carried out in an organic solvent selected from a list comprising: aliphatic alcohols such as MeOH, EtOH, iPrOH, MeCN, DMF, NMP, DMSO, ethylene or propylene carbonate, DMA, cyclic ethers such as 1,4-dioxane or THF, and aqueous mixtures thereof or water itself.

In yet another aspect of the present invention is a process, wherein the process is carried out at a temperature of 0 °C to 100 °C.

In yet another aspect of the present invention is a process, wherein the process is carried out at a temperature of 20 °C to 50 °C.

In one aspect of the present invention is a process for preparing a compound of formulae (III) or (III') 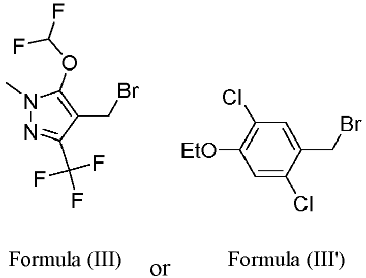 which comprises a brominating compounds of formulae (IV) or (IV'), respectively: 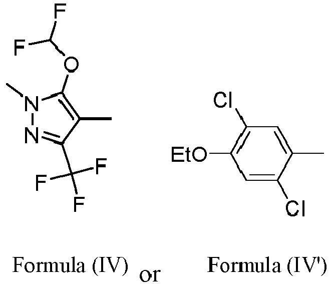 with a bromination reagent in the absence of light irradiation.

In yet another aspect of the present invention is a process, wherein the bromination reagent is selected from a list comprising: Br₂, HBr/H₂O₂ system, *N*-bromosuccinimide and 1,3-dibromo-5,5-dimethylhydantoin.

In yet another aspect of the present invention is a process, wherein the process is carried out in an organic solvent selected from a list comprising: chlorinated solvents like CH₂Cl₂, 1,2-dichloroethane, CHCl₃, CCl₄, ethylene or propylene carbonate, MeCN, water or mixtures thereof.

In yet another aspect of the present invention is a process, wherein the process is carried out at a temperature of 0 °C to 100 °C.

In yet another aspect of the present invention is a process, wherein the process is carried out at a temperature of 20 °C to 50 °C.

In yet another aspect of the present invention is a process, wherein the compound of formula I is

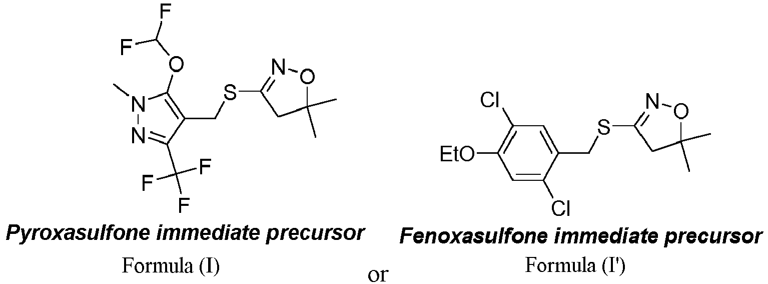

In another aspect of the present invention is a process for preparing pyroxasulfone or fenoxasulfone immediate precursors, wherein the pyroxasulfone or fenoxasulfone immediate precursors are synthesized from the arylmethyl bromide by first forming a carbon-sulfur bond at the 3-position of a 2-isoxazoline through displacement of a leaving group at the 3-position by an appropriate thionating reagent: 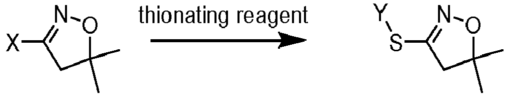 wherein Y is a protecting group and X is a leaving group.

In yet another aspect of the present invention is a process, wherein pyroxasulfone or fenoxasulfone immediate precursors are S-protected 3-thio-2-isoxazoline and are treated with a base to remove the protecting group, revealing a thiol or thiolate group: 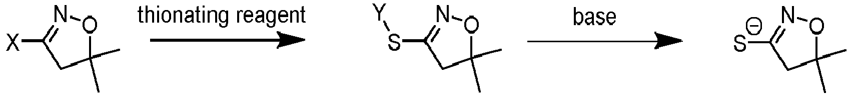 wherein Y is a protecting group and X is leaving group.

In still another aspect of the present invention is a process, wherein the pyroxasulfone or fenoxasulfone immediate precursors with a thiol or thiolate group are subsequently react with the arylmethyl bromide to form the pyroxasulfone or fenoxasulfone immediate precursor: 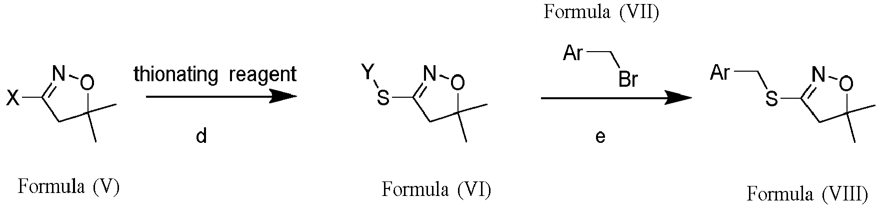 wherein Ar is aryl, Y is a protecting group and X is a leaving group.

In yet another aspect of the present invention is a process, further comprising the step of an oxidation and thereby obtaining pyroxasulfone or fenoxasulfone:

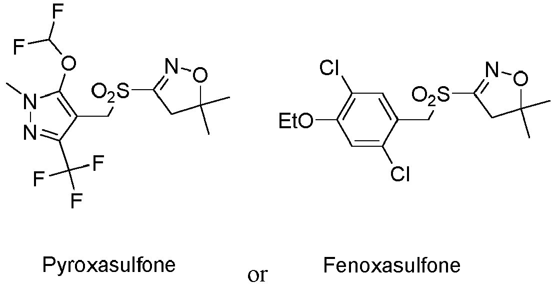

Lastly, the thioether of Formula (I) analogue undergoes oxidation to give the herbicidically active compound, i.e., pyroxasulfone, fenoxasulfone, etc. The oxidation reaction is accomplished by methods known in the art, using oxidizing agents such as organic and inorganic peroxides; operative oxidizers include hydrogen peroxide, *m-*chloroperoxybenzoic acid, peroxyacetic acid, perbenzoic acid, magnesium monoperoxyphthalate, potassium peroxymonosulfate, potassium permanganate and sodium periodate. As an alternative to direct conversion of the sulfide functionality to sulfone -SO₂-, one may consider an oxidation reaction passing through the corresponding sulfoxide (-S(O)-), i.e., with isolation of the sulfoxide and subsequent oxidation to the sulfone. The oxidation can take place in water, in organic solvent and/or in any combinations of thereof. Organic solvents such as halogenated hydrocarbons (either halogenated aliphatic hydrocarbons such as dichloromethane and chloroform or halogenated aromatic hydrocarbons such as chlorobenzene); ethers such as dioxane, tetrahydrofuran (THF), and diethyl ether; C₁-C₄ alkanols; ketones; and amides can be used. The temperature during the oxidation may vary from 0 °C to 80 °C, usually temperatures of 20 °C to 40 °C are preferred. Illustrative procedures can be found in WO 2004/013106 (=EP 1541561).

The reactants and reagents in each step can be added in succession to the reaction vessel; simultaneous feeding of two or more reactants is also workable. No requirements are placed on the order of addition.

### Definitions

Unless defined otherwise, all technical and scientific terms used herein have the meaning commonly understood by persons of ordinary skill in the art to which this subject matter pertains.

The term "alkyl" as used herein, refers to a branched, unbranched, or cyclic carbon chain, including methyl, ethyl, propyl, isopropyl, cyclopropyl and the like.

As used herein, the term "2-isoxazoline" is 5,5-dimethyl-4H-isoxazole.

As used herein, the term "S-substituted thioisoxazole" is 5,5-dimethyl-4H-isoxazole bearing a substituted sulfur atom at the 3-position.

As used herein, the term "thionating reagent" is a reagent capable of forming a new sulfur-carbon bond.

As used herein, the term "leaving group" is a molecular fragment that arises from heterolytic bond cleavage and which carries an electron pair from the bond broken during fragmentation.

In particular, the leaving group may be but is not limited to, fluoroalkylsulfinates, alkylsulfinates, arylsulfinates, chloride and bromide.

In particular, the "protecting group" may be but is not limited to, all the fragments disclosed in Figure 2, under the substituent Y.

As used herein, the term "stable" when used in connection with a composition means that the composition is physically stable and chemically stable. As used herein, the term "chemically stable" means that no significant decomposition of the active components was observed after at least 2 weeks of storage in a sealed package at a temperature of 54 °C. As used herein, the term "physically stable" means that no significant sedimentation was observed after at least 2 weeks of storage in a sealed package at a temperature of 54 °C.

The term "a" or "an" as used herein includes the singular and the plural, *unless specifically stated* otherwise. Therefore, the terms "a," "an," or "at least one" are used interchangeably in this application.

Throughout the application, descriptions of various embodiments are described using the term "comprising"; however, it will be understood by one of skill in the art, that in some specific instances, an embodiment can be described using the language "consisting essentially of" or "consisting of."

The term "about" herein specifically includes ±10 % from the indicated values in the range. In addition, the endpoints of all ranges directed to the same component or property herein are inclusive of the endpoints, are independently combinable, and include all intermediate points and ranges.

It is understood that where a parameter range is provided, all integers within that range, and tenths thereof, are also provided by the invention as if the integers and tenths thereof are expressly described herein. For example, "0.1% to 70%" includes 0.1%, 0.2%, 0.3%, 0.4%, 0.5% etc. up to 70%.

All publications, patents and patent applications mentioned in this specification are herein incorporated in their entirety by reference into the specification, to the same extent as if each individual publication, patent or patent application was specifically and individually indicated to be incorporated herein by reference.

The following examples illustrate the practice of the present subject matter in some of its embodiments but should not be construed as limiting the scope of the present subject matter. Other embodiments apparent to persons of ordinary skill in the art from consideration of the specification and examples herein that fall within the spirit and scope of the appended claims are part of this invention. The specification, including the examples, is intended to be exemplary only, without limiting the scope and spirit of the invention.

Each embodiment disclosed herein is contemplated as being applicable to each of the other disclosed embodiments. Thus, all combinations of the various elements described herein are within the scope of the invention. In addition, the elements recited in process embodiments can be used in combination with compound embodiments described herein and vice versa.

This invention will be better understood by reference to the Examples which follow, but those skilled in the art will readily appreciate that the specific experiments detailed are only illustrative of the invention as described more fully in the claims which follow thereafter.

The invention is illustrated by the following examples without limiting it thereby.

### EXAMPLES

### BROMINATION WITHOUT INITIATOR (step a.)

### Example 1: 4-(Bromomethyl)-5-(difluoromethoxy)-1-methyl-3-(trifluoromethyl)-1H pyrazole

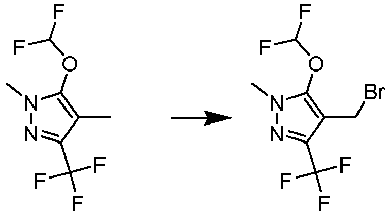

Dichloromethane (150 g) was added to a three-necked round bottom flask equipped with a condenser and connected to nitrogen flow. 5-(Difluoromethoxy)-1,4-dimethyl-3-(trifluoromethyl)-1*H*-pyrazole (10 g), HBr 48% solution (8.06) and hydrogen peroxide 28% solution (5.81) were added to the flask and the reaction mixture was purged under N₂ for 10 min. The reaction was then heated to 60°C and reflux continued overnight. A further 1.61 g of HBr 48% solution and 1.16 g of hydrogen peroxide 28% solution were added, and reflux continued for 7 h. A further 1.61 g of HBr 48% solution and 1.16 g of hydrogen peroxide 28% solution were added, and the reflux continued overnight. The reaction mixture was diluted with 20 mL water and transferred to a separatory funnel. The organic phase was separated and washed twice with 10% aqueous Na₂S₂O₃ solution (2 × 20 mL), the combined extracts dried over MgSO₄ and the solvent removed *in vacuo* to give a yellow oil, which was distilled to give 4-(bromomethyl)-5-(difluoromethoxy)-1-methyl-3-(trifluoromethyl)-1*H*-pyrazole (10.2 g, 88% purity, 76%).

### Example 2: 4-(Bromomethyl)-5-(difluoromethoxy)-1-methyl-3-(trifluoromethyl)-1H pyrazole

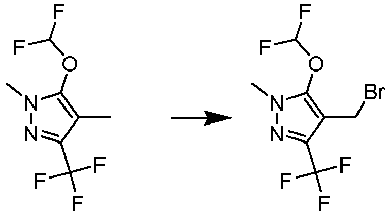

Carbon tetrachloride (30 mL) was added to a three-necked round bottom flask equipped with a condenser and connected to nitrogen flow. 5-(Difluoromethoxy)-1,4-dimethyl-3-(trifluoromethyl)-1H-pyrazole (2.16 g, 9.39 mmol) and 1,3-dibromo-5,5-dimethylhydantoin (2.69 g, 9.41 mmol) were added to the flask and the reaction mixture was purged under N₂ for 10 min. The mixture was heated to reflux (16 h). The reaction mixture was diluted with 20 mL of water and transferred to a separatory funnel. The organic phase was separated and washed twice with 10% aqueous Na₂SO₃ solution (2 × 20 mL), then the combined extracts dried over MgSO₄ and the solvent removed *in vacuo* to give 4-(bromomethyl)-5-(difluoromethoxy)-1-methyl-3-(trifluoromethyl)-1*H*-pyrazole as a yellow liquid (2.38 g, 82%).

### Example 3: 4-(Bromomethyl)-5-(difluoromethoxy)-1-methyl-3-(trifluoromethyl)-1H pyrazole

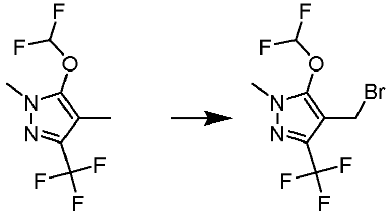

2,4-Dimethyl-5-trifluoromethyl-2*H*-pyrazol-3-ol (920 mg, 4.0 mmol, 1.0 equiv.) was taken up in CCl₄ (20 mL) and the resulting mixture sparged with nitrogen for 10 minutes with vigorous stirring. Sparging and stirring were continued as 1,3-dibromo-5,5-dimethylhydantoin (572 mg, 2.0 mmol, 0.5 equiv.) then AIBN (100 mg, 0.41 mmol, 0.10 equiv.) were added. The mixture was heated to reflux (3 h) then poured onto ice (100 g) and 5% aqueous sodium sulfite (100 mL) added. The mixture was extracted with CH₂Cl₂ (3 × 20 mL) and the combined organic extracts washed with water (100 mL), brine (50 mL), passed through a plug of silica, washing with CH₂Cl₂ (50 mL) and concentrated to give 4-(bromomethyl)-5-(difluoromethoxy)-1-methyl-3-(trifluoromethyl)-1*H*-pyrazole as a slightly yellow oil (891 mg, 84%).

### ARYLMETHYL BROMIDE REACTION WITH THIONATING AGENT (step b.)

### Example 4: S-((5-(Difluoromethoxy)-1-methyl-3-(trifluoromethyl)-1H pyrazol-4-yl)methyl) ethanethioate

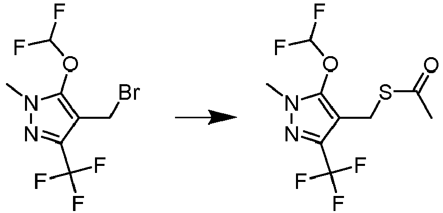

4-(bromomethyl)-5-(difluoromethoxy)-1-methyl-3-(trifluoromethyl)-1*H*-pyrazole (0.50 g) was taken up in MeOH (10 mL) and a solution of KSAc (0.28 g) in water (5 mL) added. After stirring for 0.5 h, MeOH (5 mL) was removed *in vacuo* and the resulting mixture extracted with ethyl acetate (2 × 10 mL), the combined extracts dried over MgSO₄ and the solvent removed *in vacuo* to give S-((5-(difluoromethoxy)-1-methyl-3-(trifluoromethyl)-1*H*-pyrazol-4-yl)methyl) ethanethioate (0.47 g, 94.1% purity, 90%).

### STEP B AND C PERFORMED TOGETHER

### Example 5: 3-(5-(Difluoromethoxy)-1-methyl-3-(trifluoromethyl)-1H-pyrazol-4-yl)methylsulfanyl-4H-5,5-dimethylisoxazole

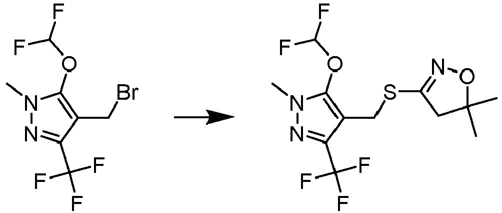

4-(bromomethyl)-5-(difluoromethoxy)-1-methyl-3-(trifluoromethyl)-1*H*-pyrazole (0.50 g) was taken up in MeOH (10 mL) KSAc (0.28 g) added. After stirring for 0.5 h the mixture was filtered and the filtrate added over 1 hour to a mixture of potassium carbonate (0.55 g) and 3-bromo-5,5-dimethyl-4H-isoxazole (0.35 g) at reflux. Reflux was continued for a further 3 hours then the methanol solvent removed in vacuo at 40°C. The mixture was partitioned between water (10 mL) and methyl tert-butyl ether (10 mL) by vigorous stirring at ambient temperature (0.5 h) and the methyl tert-butyl ether fraction concentrated to give an orange oil 3-(5-(difluoromethoxy)-1-methyl-3-(trifluoromethyl)-1*H*-pyrazol-4-yl)methylsulfanyl-4H-5,5-dimethylisoxazole (1.67 g, 15.3% purity, 44%).

### ARYLMETHYL BROMIDE WITH S-PROTECTED 3-THIO-2-ISOXAZOLINE

### Example 6: 3-(5-(Difluoromethoxy)-1-methyl-3-(trifluoromethyl)-1H pyrazol-4-yl)methylsulfanyl-4H 5,5-dimethylisoxazole

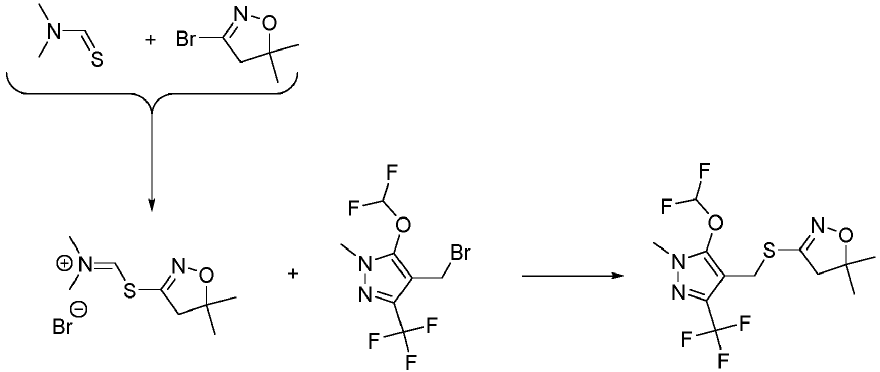

Acetonitrile (20 mL) was sparged with nitrogen (10 minutes), with stirring. Dimethylthioformamide (0.706 g) was added, followed by trifluoroacetic acid (30 µL) and then 3-bromo-5,5-dimethyl-4H-isoxazole (1.41 g) was added over 5 minutes, at ambient temperature. This mixture was stirred (16 h), under a nitrogen atmosphere.

Separately, potassium carbonate (2.7 g) was taken up in methanol (20 mL) and the mixture heated to 50°C, with stirring. 4-(Bromomethyl)-5-(difluoromethoxy)-1-methyl-3-(trifluoromethyl)-1H-pyrazole (2.0 g) was added, then the previously prepared solution of dimethylthioformamide/3-bromo-5,5-dimethyl-4H-isoxazole adduct in acetonitrile was added over 0.5 h at 50°C. Stirring was continued at 50°C (2 h) then the reaction mixture concentrated in vacuo to an approximate weight of 10 g and water (50 mL) added. The resulting mixture was extracted with ethyl acetate (2 × 20 mL) and the ethyl acetate extracts combined and the solvent removed in vacuo to give 3-(5-(difluoromethoxy)-1-methyl-3-(trifluoromethyl)-1H-pyrazol-4-yl)methylsulfanyl-4H-5,5-dimethylisoxazole as an orange oil (2.3 g, 79% purity, 78%).

## Claims

1. A process for preparing pyroxasulfone or fenoxasulfone immediate precursors of formulae (I) or (I') and various sulfone analogs of 5,5-dimethyl-4H-1,2-oxazole, which comprises the following steps:
d) reacting an isoxazole of Formula (V) with a thionating reagent to create an S-substituted thioisoxazole of Formula (VI); 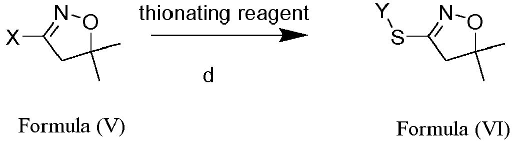 wherein X is a leaving group and Y is a protecting group;
e) substitution of the resulting thiol or thiolate created from formula (VI) compounds of step d with arylmethyl-bromide compounds of formula (VII) to yield the compound of formula (VIII)

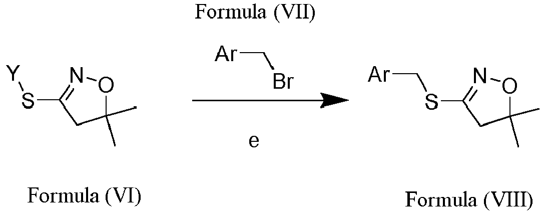

2. A process according to claim 1, wherein pyroxasulfone or fenoxasulfone immediate precursors are S-protected 3-thio-2-isoxazoline and are treated with a base to remove the protecting group, revealing a thiol or thiolate group: 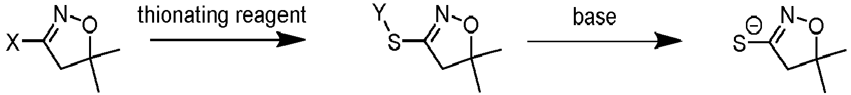 wherein Y is a protecting group and X is a leaving group.

3. A process according to claim 2, wherein the pyroxasulfone or fenoxasulfone immediate precursors with a thiol or thiolate group are subsequently react with the arylmethyl bromide to form the pyroxasulfone or fenoxasulfone immediate precursor: 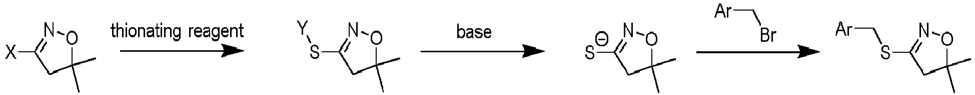 wherein Ar is aryl, Y is a protecting group and X is a leaving group.

4. A process according to any one of the preceding claims, further comprising the step of an oxidation and thereby obtaining pyroxasulfone or fenoxasulfone:
